# EUROPÄISCHE PATENTSCHRIFT

(11) **EP 0 900 572 B1**
(45) Veröffentlichungstag und Bekanntmachung des Hinweises auf die Patenterteilung: **12.01.2005**
(21) Anmeldenummer: 97810629.2
(22) Anmeldetag: 04.09.1997
(51) Int. Cl.: A61M 1/10, F04D 13/06, F04D 29/24, F04D 29/44

(54) **Zentrifugalpumpe**
Centrifugal pump
Pompe centrifuge

(43) Veröffentlichungstag der Anmeldung: 10.03.1999
(73) Patentinhaber: Levitronix LLC, Waltham, MA 02451 (US)
(72) Erfinder: Schöb, Reto, Dr., 8604 Volketswil (CH)
(74) Vertreter: Sulzer Management AG

(56) Entgegenhaltungen:
- EP-A- 0 447 106
- WO-A-93/20860
- WO-A-96/31934
- AT-B- 395 944
- DE-C- 924 188
- FR-A- 1 276 208
- FR-A- 2 102 520
- US-A- 4 213 742
- US-A- 4 984 972
- US-A- 5 601 418

## Beschreibung

Die Erfindung betrifft eine Zentrifugalpumpe zum Fördern eines Fluids gemäss dem Oberbegriff des unabhängigen Patentanspruchs. Insbesondere betrifft die Erfindung eine Zentrifugalpumpe zum Fördern von Blut und im speziellen eine Zentrifugalpumpe, bei der das Flügelrad magnetisch gelagert ist und durch ein Drehfeld angetrieben wird.

In der WO-A-96/31934 wird eine Rotationspumpe offenbart, bei der das Flügelrad im Innern des Pumpengehäuses durch magnetische Kräfte schwebend gelagert ist und durch ein Drehfeld angetrieben wird, das von einem ausserhalb des Pumpengehäuses angeordneten Stator erzeugt wird. Solche Pumpen sind insbesondere für solche Anwendungen vorteilhaft, bei denen das zu fördernde Fluid nicht verunreinigt werden darf, beispielsweise zum Fördern biologischer Flüssigkeiten wie Blut oder hochreiner Flüssigkeiten wie Reinstwasser. Zudem eignen sich solche Rotationspumpen zum Fördern aggressiver Flüssigkeiten, die mechanische Lager in kurzer Zeit zerstören würden.

Bei der Ausgestaltung solcher Rotationspumpen als Zentrifugalpumpen besteht das Problem, dass sich die Wickelköpfe des Stators und der Auslass des Pumpengehäuses räumlich behindern. Zur Lösung dieses Problems wird in der WO-A-96/31934 (siehe z. B. Fig. 12) eine Pumpe mit einem sogenannten Tempelmotor vorgeschlagen, bei dem die Spulenkerne des Stators jeweils die Form eines "L" haben, wobei der lange Schenkel jeweils parallel zur Rotationsachse verläuft, während der kurze Schenkel radial einwärts zur Rotationsachse gerichtet ist. Der Stator, der als Lagerund Antriebsstator ausgestaltet ist, hat zwei Wicklungen, nämlich die Antriebswicklung und die Steuerwicklung, welche als diskrete Spulen ausgeführt sind und um die langen Schenkel der L-förmigen Spulenkerne gewickelt sind. Ein solcher Tempelmotor kommt ohne Wickelköpfe aus, sodass der Auslass des Pumpengehäuses ohne räumliche Behinderung auf der Höhe des Flügelrads angeordnet werden kann. Diese Ausgestaltung als Tempelmotor unterliegt jedoch der Einschränkung, dass sie einen relativ grossen Platbedarf aufweist und relativ aufwendig ist.

Es ist die Aufgabe der vorliegenden Erfindung, eine Zentrifugalpumpe bereitzustellen, die sehr kompakt ausgestaltet ist und dabei eine hohe Förderleistung ermöglicht. Die Zentrifugalpumpe soll möglichst wenig aufwendig sein und insbesondere eine Ausgestaltung ermöglichen, bei der das Flügelrad magnetisch gelagert und durch ein elektomagnetisches Drehfeld antreibbar ist. Im speziellen ist es eine Aufgabe der Erfindung eine möglichst kompakte und leistungsfähige Zentrifugalpumpe bereitzustellen, die gemäss dem Prinzip des lagerlosen Motors betreibbar ist.

Die diese Aufgabe lösende Zentrifugalpumpe ist durch die Merkmale des unabhängigen Anspruchs gekennzeichnet.

Weitere vorteilhafte Massnahmen und Ausgestaltungen ergeben sich aus den abhängigen Ansprüchen.

Die Erfindung betrifft somit eine Zentrifugalpumpe zum Fördern eines Fluids, insbesondere zum Fördern von Blut, mit einem Pumpengehäuse mit Gehäusedeckel, wobei das Pumpengehäuse einen Einlass sowie einen Auslass für das Fluid aufweist. Im Innern des Pumpengehäuses ist ein Flügelrad angeordnet, das mehrere Flügel aufweist und um eine Rotationsachse rotierbar ist, sowie mit Mitteln zum Antreiben des Flügelrads, welche Mittel zum Antreiben einen passiven magnetisch wirksamen Rotor umfassen, der Teil des Flügelrads ist, sowie einen das Pumpengehäuse umgebenden Stator, der elektrische Wicklungen aufweist, wobei der Stator als Lager- und Antriebsstator ausgestaltet ist, sodass das Flügelrad im Betriebszustand durch die magnetische Wechselwirkung zwischen dem Stator und dem Rotor sowohl antreibbar als auch berührungslos in dem Pumpengehäuse lagerbar ist. Dabei ist der Auslass bezüglich des Flügelrads in axialer Richtung versetzt angeordnet und ist in dem Pumpengehäuse ein im wesentlichen ringförmiger, in Umfangsrichtung des Pumpengehäuses verlaufenden Leitkanal vorgesehen der den Einlass mit dem Auslass verbindet. Die Flügel des Flügelrads erstrecken sich in radialer Richtung gesehen bis in den Leitkanal hinein. Dabei ist eine in axialer Richtung zwischen den Flügeln des Flügelrads und dem Gehäusedeckel des Pumpengehäuses angeordnete Deckplatte vorgesehen, die sich in radialer Richtung bis an die radial innere Begrenzung des Leitkanals erstreckt, wobei damit gemeint ist, dass sich die Deckplatte in radialer Richtung ungefähr bis an die radial innere Begrenzung des Leitkanals erstreckt.

Im folgenden wird die Erfindung anhand von Ausführungsbeispielen und anhand der Zeichnung näher erläutert. In der schematischen nicht massstäblichen Zeichnung, in der identische oder von der Funktion her gleichwertige Teile mit den gleichen Bezugszeichen versehen sind, zeigen:
- Fig. 1:: einen Längsschnitt durch ein erstes Ausführungsbeispiel der erfindungsgemässen Zentrifugalpumpe,
- Fig. 2:: einen Querschnitt durch das erste Ausführungsbeispiel entlang der Schnittlinie II-II in Fig. 1,
- Fig. 3:: einen Querschnitt durch das erste Ausführungsbeispiel entlang der Schnittlinie III-III in Fig. 1,
- Fig. 4:: einen Längsschnitt durch eine Weiterentwicklung des ersten Ausführungsbeispiels,
- Fig. 5:: einen Querschnitt analog zu Fig. 3 für eine Variante der Ausgestaltung des Leitkanals (Stator und elektrische Wicklungen nicht dargestellt),
- Fig. 6:: einen Längsschnitt durch ein zweites Ausführungsbeispiel der erfindungsgemässen Zentrifugalpumpe,
- Fig. 7:: einen Querschnitt durch das zweite Ausführungsbeispiel entsprechend der Fig. 3,
- Fig. 8:: ein Diagramm, welches die axiale Höhe des Leitkanals als Funktion eines Azimutwinkels zeigt,
- Fig. 9:: einen Längsschnitt durch eine Weiterentwicklung des zweiten Ausführungsbeispiels,
- Fig. 10:: eine Detaildarstellung des Rotors aus Fig. 9,
- Fig. 11:: eine Aufsicht auf das Flügelrad aus Fig. 9, vom Einlass her in axialer Richtung (Deckplatte nicht dargestellt),
- Fig. 12:: eine Aufsicht auf das Flügelrad vom Einlass her in axialer Richtung für eine Variante der Flügel (Deckplatte nicht dargestellt), und
- Fig. 13:: eine Aufsicht auf das Flügelrad vom Einlass her in axialer Richtung für eine weitere Variante der Flügel (Deckplatte nicht dargestellt).

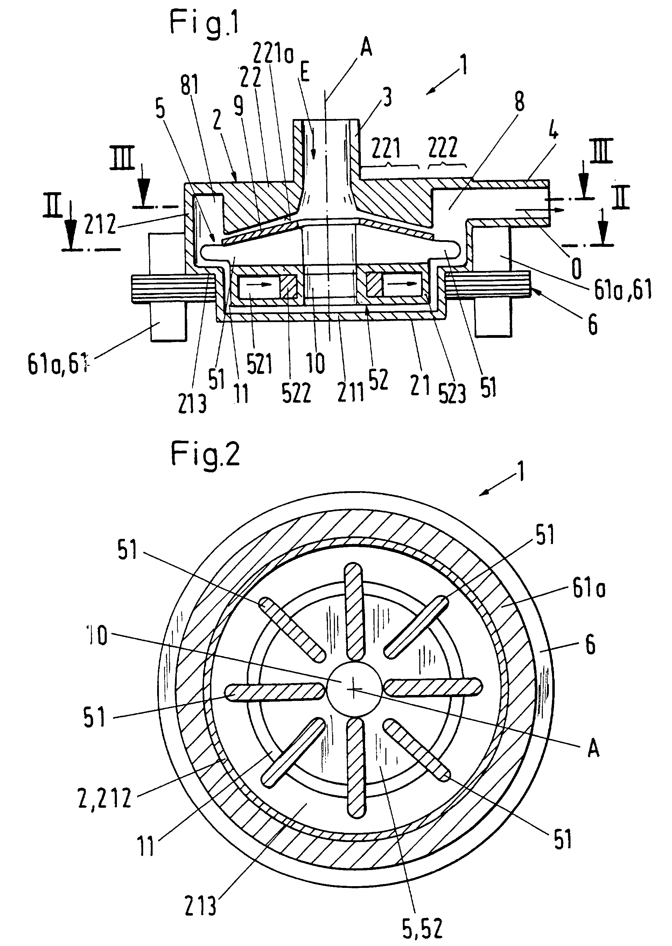

Die erfindungsgemässe Zentrifugalpumpe zum Fördern eines Fluids, insbesondere zum Fördern von Blut, hat ein Pumpengehäuse 2 (Fig. 1), das einen Einlass 3 sowie einen Auslass 4 für das Fluid aufweist, sowie ein im Innern des Pumpengehäuses 2 angeordneten Flügelrad 5, das mehrere Flügel 51 aufweist und um eine Rotationsachse A rotierbar ist. Ferner sind Mittel zum Antreiben des Flügelrads 5 vorgesehen. Bei der erfindungsgemässen Zentrifugalpumpe ist der Auslass 4 bezüglich des Flügelrads 5 in axialer Richtung versetzt angeordnet, wobei der Einlass 3 und Auslass 4 mittels eines ringförmigen Leitkanals 8 verbunden sind. Eine Deckplatte 9 des Flügelrads 5 erstreckt sich radial ungefähr bis an die innere Begrenzung des Leitkanals 8. Die Flügel 51 erstrecken sich bis in den Leitkanal 8.

Mit Zentrifugalpumpen sind solche Rotations- oder Kreiselpumpen gemeint, bei denen das zu fördernde Fluid in Richtung der Rotationsachse A zu dem Flügelrad strömt und das Pumpengehäuse 2 in radialer oder tangentialer Richtung verlässt.

Bei einer besonders bevorzugten Ausgestaltung der erfindungsgemässen Zentrifugalpumpe wird das Flügelrad 5 magnetisch und berührungslos im Pumpengehäuse 2 gelagert und durch ein elektomagnetisches Drehfeld angetrieben.

Die Mittel zum Antreiben umfassen dabei einen passiven magnetisch wirksamen Rotor 52, der Teil des Flügelrads 5 ist, sowie einen das Pumpengehäuse 2 umgebenden Stator 6, der elektrische Wicklungen 61 aufweist. Der Stator 6 ist vorzugsweise als Lager- und Antriebsstator ausgestaltet, sodass das Flügelrad 5 im Betriebszustand durch die magnetische Wechselwirkung zwischen dem Stator 6 und dem Rotor 52 sowohl antreibbar als auch berührungslos in dem Pumpengehäuse 2 lagerbar ist. Vorzugsweise bilden der Stator 6 und der Rotor 52 einen sogenannten lagerlosen Motor, der in analoger Weise ausgestaltet sein kann wie dies in der WO-A-96/31934 und insbesondere auch in der internationalen Anmeldung WO-A-98/11650 offenbart ist. Die Funktionsweise bezüglich Antrieb und magnetischer Lagerung des Rotors 52 sind in diesen beiden Druckschriften, deren Inhalt hiermit in diese Beschreibung inkorporiert wird, detailiert beschrieben und werden daher hier nicht näher erläutert.

Im folgenden werden nur kurz die wesentlichsten Merkmale des Prinzips des lagerlosen Motors zusammengefasst. Unter einem lagerlosen Motor wird eine elektrisch ansteuerbare Lager- und Antriebsvorrichtung verstanden, welche den Stator 6 und den Rotor 52 umfasst. Der Begriff "lagerloser Motor" bringt zum Ausdruck, dass der Stator 6 gleichzeitig Antriebs- und Lagerstator ist, also kein separater Stator (und auch kein separater Rotor) für die magnetische Lagerung vorgesehen ist. Der Stator 6 ist so ausgestaltet bzw. mit elektrischen Wicklungen 61 versehen, dass er ein elektromagnetisches Drehfeld erzeugt, welches zum einen ein Drehmoment auf den Rotor 52 ausübt, dass dessen Rotation um die Rotationsachse A antreibt, und welches zum anderen eine beliebig einstellbare Querkraft auf den Rotor ausübt, sodass dessen Position bezüglich einer zur Rotationsachse A senkrechten Ebene vorgebbar bzw. aktiv steuerbar ist. Somit ist der Rotor 52 im Betriebszustand mittels der elektrischen Wicklungen 61 des Stators 6 bezüglich dreier Freiheitsgrade, nämlich der Rotation um die Rotationsachse A sowie der Position bezüglich der zur Rotationsachse A senkrechten Ebene (zwei Freiheitsgrade) aktiv ansteuerbar bzw. antreibbar. Dazu umfassen die elektrischen Wicklungen 61 des Stators 6 beispielsweise eine Antriebswicklung mit der Polpaarzahl p und eine Steuerwicklung mit der Polpaarzahl p+1 oder p-1.

Bezüglich dreier weiterer Freiheitsgrade, nämlich Verkippungen bezüglich der zur Rotationsachse A senkrechten Ebene (zwei Freiheitsgrade) und axiale Position, ist der Rotor 52 vorzugsweise durch Reluktanzkräfte magnetisch passiv, das heisst nicht ansteuerbar, im Stator 6 gelagert. Dazu weist der Stator 6 z. B. mehrere radial nach innen weisende Zähne auf (nicht dargestellt). Für weitere Details der Ausgestaltung und der Ansteuerung des Stators 6 bzw. der Ausgestaltung des Rotors 52 sei hier auf die WO-A-96/31934 und insbesondere auch auf die internationalen Anmeldung WO-A-98/11650 verwiesen. Die dort zu findenden Erläuterungen bezüglich Antrieb und Lagerung lassen sich in analoger Weise auch für die erfindungsgemässe Zentrifugalpumpe verwenden.

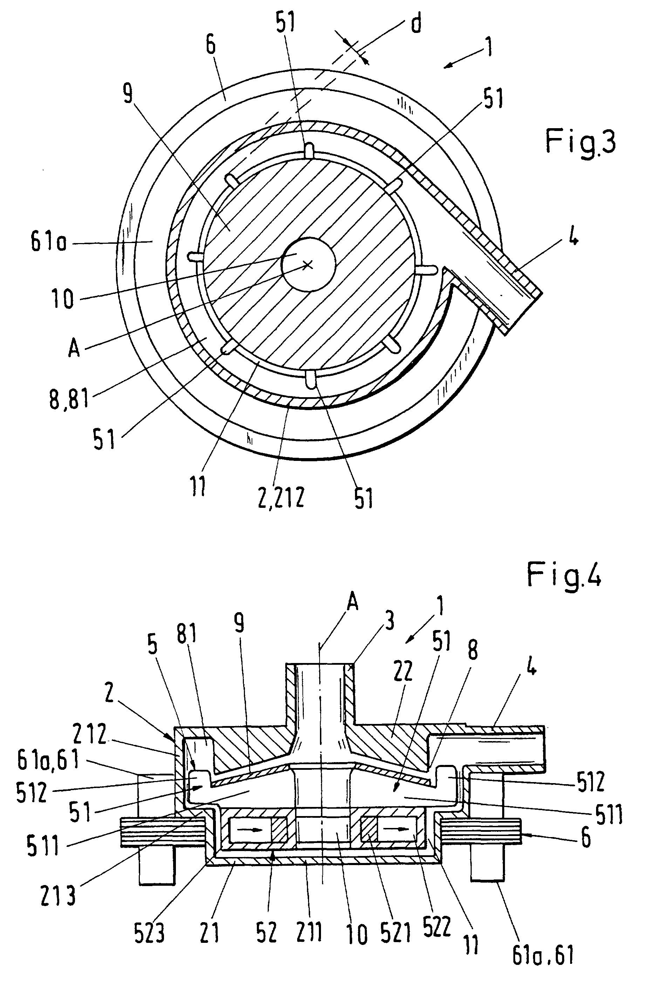

Fig. 1 zeigt in einer Längsschnittdarstellung ein erstes Ausführungsbeispiel der erfindungsgemässen Zentrifugalpumpe, die gesamthaft mit dem Bezugszeichen 1 versehen ist. Zum besseren Verständnis zeigen die Figuren 2 und 3 dieses Ausführungsbeispiel jeweils in einer Querschnittsdarstellung entlang der Schnittlinie II-II (Fig. 2) bzw. entlang der Schnittlinie III-III (Fig. 3) in Fig. 1. Das Pumpengehäuse 2 umfasst einen Gehäusedeckel 22 sowie ein Gehäuseteil 21, die hermetisch dicht miteinander verbindbar sind. Der Einlass 3 ist als axialer Einlass ausgestaltet, durch den das zu fördernde Fluid in das Pumpengehäuse 2 gelangt, wie dies der Pfeil E andeutet. Der Auslass 4 ist als tangentialer Auslass angeordnet, durch den das Fluid das Pumpengehäuse 2 verlässt, wie dies der Pfeil O andeutet. Der Einlass 3 und der Auslass 4 sind mit nicht dargestellten Leitungen bzw. Schläuchen für das Fluid verbunden. Im Innern des Pumpengehäuses 2 ist das Flügelrad 5 angeordnet, das den scheiben- oder ringförmigen Rotor 52 umfasst, sowie mehrere auf dem Rotor 52 angeordnete Flügel 51, die vorzugsweise aus Kunststoff hergestellt sind. Aus Gründen der besseren Übersicht sind in Fig. 1 nur zwei der Flügel dargestellt. Im Betriebszustand rotiert das Flügelrad 5 um die Rotationsachse A. Das Pumpengehäuse 2 ist von dem Lager- und Antriebsstator 6 mit den elektrischen Wicklungen 61 umgeben. Die elektrischen Wicklungen 61 sind als Wickelköpfe 61a dargestellt. Der ringförmige Rotor 52 umfasst einen ringförmigen Permanentmagnet 521, einen dazu koaxial und radial innenliegend angeordneten Eisenrückschluss 522 sowie eine Ummantelung 523, die vorzugsweise aus Titan oder Kunststoff hergestellt ist. Die Flügel 51 sind drehfest mit der Ummantelung 523 verbunden. Der Permanentmagnet 521 ist beispielsweise diametral magnetisiert wie dies die beiden Pfeile ohne Bezugszeichen andeuten. Der passive magnetisch wirksame Rotor 52 bildet zusammen mit dem Stator 61 und nicht dargestellten Versorgungs- und Steuereinrichtungen einen lagerlosen Motor, wobei der Rotor 52 bezüglich der Rotation um die Achse A sowie seiner Position in der zur Rotationsachse A senkrechten Ebene aktiv ansteuerbar bzw. antreibbar ist und hinsichtlich Verkippungen relativ zu genannter Ebene sowie seiner axialen Position magnetisch passiv durch Reluktanzkräfte im Stator 6 gelagert ist.

Wie insbesondere aus Fig. 1 ersichtlich, ist erfindungsgemäss der Auslass 4 bezüglich des Flügelrads 5 in axialer Richtung versetzt, nämlich gemäss der Darstellung in Fig. 1 höher, angeordnet. Durch diese Massnahme lassen sich in sehr einfacher Weise die konstruktiven Probleme lösen, die daraus resultieren, dass bei der Zentrifugalpumpe der Auslass 4 an den Wickelköpfen 61a des Stators 6 vorbei geführt werden muss. Der Auslass 4 wird nämlich -gemäss der Darstellung in Fig. 1 - oberhalb der Wickelköpfe 61a vorgesehen, sodass es zu keiner räumlichen Behinderung zwischen dem Auslass 4 und den Wickelköpfen 61a kommt. Aus Gründen der Kompaktheit wird der Auslass 4 vorzugsweise unmittelbar über den Wickelköpfen 61a angeordnet und liegt insbesondere auf ihnen auf. Im Vergleich zu dem eingangs erwähnten Tempelmotor weist die erfindungsgemässe Anordnung des Auslasses 4 den Vorteil auf, dass sie eine axial deutlich kürzere Bauart der Zentrifugalpumpe 1 zulässt, die somit wesentlich kompakter ausgestaltet werden kann.

Das Gehäuseteil 21 bildet den im wesentlichen ebenen Boden 211 des Pumpengehäuses 2 sowie dessen Seitenwand 212. In der Seitenwand 212 ist ein Gehäuseabsatz 213 vorgesehen, an dem sich das Pumpengehäuse 2 in radialer Richtung erweitert. Der Gehäusedeckel 22 hat einen zentralen Bereich 221, dessen innere dem Flügelrad 5 zugewandte Begrenzungsfläche 221a konisch ausgestaltet ist und der sich in radialer Richtung ungefähr gleich weit erstreckt wie der Boden 211. An den zentralen Bereich 221 schliesst sich in radialer Richtung ein im wesentlichen ringförmiger Bereich 222 an, der sich bis zu der Seitenwand 212 erstreckt und dessen axiale Dicke kleiner ist als die des zentralen Bereichs 221. Somit weist das Pumpengehäuse 2 einen im wesentlichen ringförmigen in Umfangsrichtung des Pumpengehäuses 2 verlaufenden Leitkanal 8 auf, der den Einlass 3 mit dem Auslass 4 verbindet. Der Leitkanal 8 wird begrenzt durch den Gehäuseabsatz 213, die Seitenwand 212, die Wandung des ringförmigen Bereichs 222 und die seitliche Begrenzung des zentralen Bereichs 221. Der Leitkanal 8 umfasst somit einen Teilraum 81, der in axialer Richtung gesehen bezüglich des Flügelrads 5 versetzt angeordnet ist, nämlich denjenigen Teilraum, der den zentralen Bereich 221 des Gehäusedeckels 22 in Umfangsrichtung umgibt und gemäss der Darstellung in Fig. 1 oberhalb der radial äusseren Enden der Flügel 51 liegt. Diese Ausgestaltung des Leitkanals 8 kann bildlich gesprochen so beschrieben werden, dass der Leitkanal, der bei konventionellen Zentrifugalpumpen symmetrisch bezüglich der äusseren Enden der Flügel angeordnet ist, in die axiale Richtung - gemäss Fig. 1 nach oben - "gefaltet" ist.

Da somit der Leitkanal 8 durch den axial versetzten Teilraum 81 ausreichend Volumen für das zu fördernde Fluid aufweiset, sind die Flügel 51 des Flügelrads 5 so ausgestaltet, dass sie sich in radialer Richtung gesehen bis in den Leitkanal 8 hinein erstrecken. Durch diese Massnahme vergrössert sich nämlich die effektiv wirksame Flügellänge, wodurch sich die Leistungsfähigkeit, insbesondere die Förderleistung, der Zentrifugalpumpe 1 noch steigern lässt, ohne dass sich die äusseren Abmessungen der Zentrifugalpumpe 1 vergrössern. Diese Ausgestaltung der Flügel 51, die in radialer Richtung über den Rotor 52 hinausragen, beispielsweise um 15%-20% seines Durchmessers, ist insbesondere in den Fig. 2 und 3 deutlich zu erkennen.

Wie dies am besten aus der Querschnittsdarstellung in Fig. 3 ersichtlich ist, ist der Leitkanal 8 bei diesem Ausführungsbeispiel als Ringraum ausgestaltet, das heisst mit einem Querschnitt, der über seinen Umfang im wesentlichen gleichbleibend ist, sodass alle radial äussern Enden der Flügel 51 im normalen Betriebszustand im wesentlichen den gleichen Abstand d zur Seitenwand 212 des Pumpengehäuses 2 haben.

In axialer Richtung ist zwischen den Flügeln 51 des Flügelrads 5 und dem Gehäusedeckel 22 des Pumpengehäuses 2, speziell der konischen Begrenzungsfläche 221a des zentralen Bereichs 221 des Gehäusedeckels 22, noch eine ebenfalls konische Deckplatte 9 ( siehe auch Fig. 1) angeordnet, z. B. auf die Flügel 51 aufgesetzt , um die Rückstömung des Fluids aus dem Leitkanal 8 in Richtung des Einlasses 3 zu reduzieren. Die Deckplatte 9 erstreckt sich in radialer Richtung bis an die radial innere Begrenzung des Leitkanals 8, also die seitliche Begrenzung des zentralen Bereichs 221 des Gehäusedeckels 22, sodass die Flügel 51 in radialer Richtung gesehen über das Ende der Deckplatte hinaus ragen.

Das zentrale Loch des ringförmigen Rotors 52 stellt in dem Ausführungsbeispiel gemäss Fig. 1-3 eine durchgängige Entlastungsbohrung 10 des Flügelrads 5 in seinem axialen Bereich dar. Im Betriebszustand der Zentrifugalpumpe 1 kann das Fluid durch den Spalt 11 zwischen dem Flügelrad 5 und der Seitenwand 212 in den Raum zwischen dem Boden 211 und der ihm zugewandten Endläche des Rotors 52 gelangen und von dort in axialer Richtung durch die Entlastungsbohrung 10 zurückströmen. Durch eine entsprechende Dimensionierung des Spalts 11 und der Entlastungsbohrung 10 lässt sich somit ein hydrodynamischer Ausgleich des Axialschubs erzielen, welcher die in axialer Richtung, gemäss der Darstellung in Fig. 1 nach oben wirkenden Kräfte auf das Flügelrad 5 zumindest teilweise kompensiert. Durch diese Massnahme reicht die passive magnetische Axiallagerung des Flügelrads 5 insbesondere auch bei hohen Förderleistungen aus.

Im Betriebszustand rotiert das Flügelrad 5, angetrieben und in der Schwebe gehalten durch die magnetische Wechselwirkung zwischen dem Rotor 52 und dem Lager- und Antriebsstator 6, um die Rotationsachse A. Das zu fördernde Fluid gelangt in axialer Richtung durch den Einlass 3, wird durch die Flügel 51 in im wesentlichen radialer Richtung beschleunigt und in den Leitkanal 8 gefördert, wo es zunächst in die axiale Richtung umgelenkt wird und dann den Leitkanal durch den Auslass 4 in tangentialer Richtung verlässt. Dabei herrscht im Leitkanal 8 zumindest eine Strömungskomponente, die der Strömung im Bereich des Einlasses 3 entgegengesetzt gerichtet ist.

Fig. 4 zeigt einen Längsschnitt durch eine Weiterentwicklung des ersten Ausführungsbeispiels. Auch hier sind aus Gründen der besseren Übersicht nur zwei der Flügel 51 dargestellt. Bei dieser Weiterentwicklung sind die Flügel 51 des Flügelrads 5 jeweils gewinkelt ausgestaltet, sodass sie jeweils einen Radialteil 511 aufweisen, der sich geradlinig oder gekrümmt in radialer Richtung nach aussen erstreckt, sowie einen daran anschliessenden Axialteil 512, der sich im wesentlichen parallel zur Rotationsachse A des Flügelrads 5 bzw. parallel zur Seitenwand 212 des Pumpengehäuses 2 ersreckt. Der Axialteil 512 reicht bis in den Teilraum 81 des Leitkanals 8 hinein. Die Flügel 51 sind also jeweils in die axiale Richtung, gemäss der Darstellung in Fig. 4 nach oben, "gefaltet". Durch diese vorteilhafte Massnahme wird die effektiv wirksame Flügellänge noch weiter vergrössert, wodurch sich die Leistungsfähigkeit, insbesondere die Förderleistung, der Zentrifugalpumpe nochmals steigern lässt, ohne dass sich die äusseren Abmessungen der Zentrifugalpumpe vergrössern. Insbesondere wird durch die gefaltete Ausgestaltung der Flügel 51 bewirkt, dass das Fluid länger durch die Flügel 51 geführt wird, wodurch sich der Druck des Fluids erhöhen lässt.

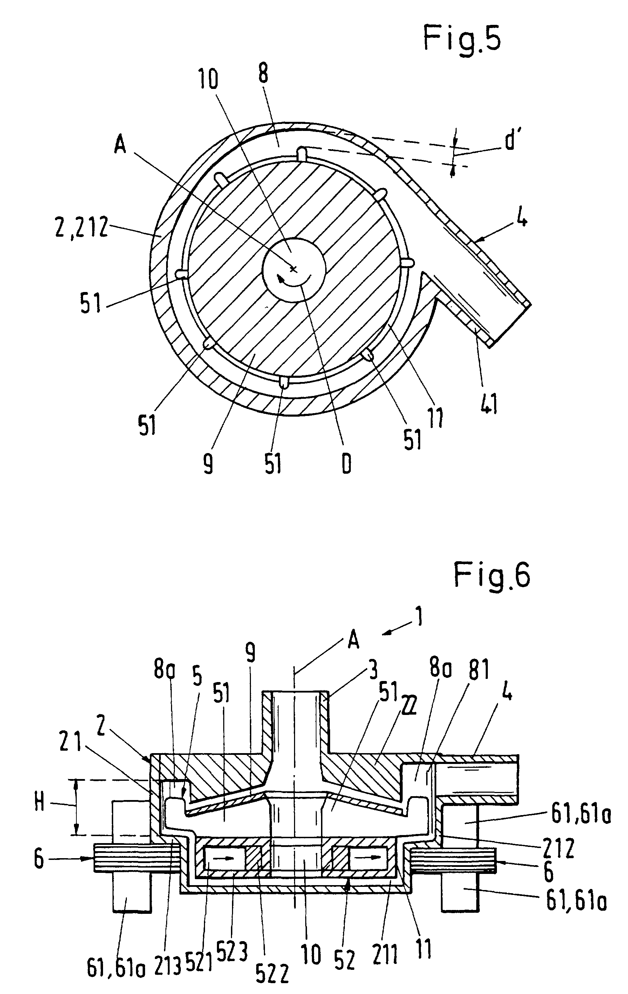

In Fig. 5 ist eine Variante für die Ausgestaltung des Leitkanals 8 verdeutlicht. Fig. 5 entspricht von der Darstellung her der Fig. 3, also einem Querschnitt entlang der Schnittlinie III-III in Fig. 1. Auf die Darstellung des Stators 6 und der elektrischen Wicklungen 61 wurde jedoch in Fig. 5 verzichtet, weil sie analog wie in Fig. 3 gezeigt angeordnet sind.

Bei dieser Variante gemäss Fig. 5 ist der Leitkanal 8 in radialer Richtung als Spiralraum ausgestaltet, sodass der Abstand d' zwischen den radial äusseren Enden der Flügel 51 und der den Leitkanal 8 begrenzenden Wandung des Pumpengehäuses, nämlich der Seitenwand 212, in Rotationsrichtung gesehen zunimmt. Die Rotationrichtung ist in Fig. 5 durch den Pfeil D angedeutet. Der Abstand d' ist im Bereich des kürzeren Schenkels 41 des tangentialen Auslasses 4 am geringsten und nimmt dann in Rotationsrichtung D gesehen zu. Diese Ausgestaltung des Leitkanals 8 als radialer Spiralraum trägt der Tatsache Rechnung, dass das Volumen des im Leitkanal befindlichen Fluids in Rotationsrichtung D gesehen zum Auslass 4 hin zunimmt. Der radiale Spiralraum lässt sich z. B realisieren, indem die Seitenwand 212 des Pumpengehäuses 2 in Umfangsrichtung eine abnehmende Wandstärke aufweist, wie dies aus Fig. 5 ersichtlich ist.

Fig. 6 zeigt in einer Längsschnittdarstellung ein zweites Ausführungsbeispiel der erfindungsgemässen Zentrifugalpumpe 1. Auch in Fig. 6 sind aus Gründen der besseren Übersicht nur zwei der Flügel 51 dargestellt. Der wesentliche Unterschied zum ersten Ausführungsbeispiel besteht in der Ausgestaltung des Leitkanals 8a, ansonsten gelten die Erläuterungen des ersten Ausführungsbeispiels in sinngemäss gleicher Weise auch für das zweite Ausführungsbeispiel.

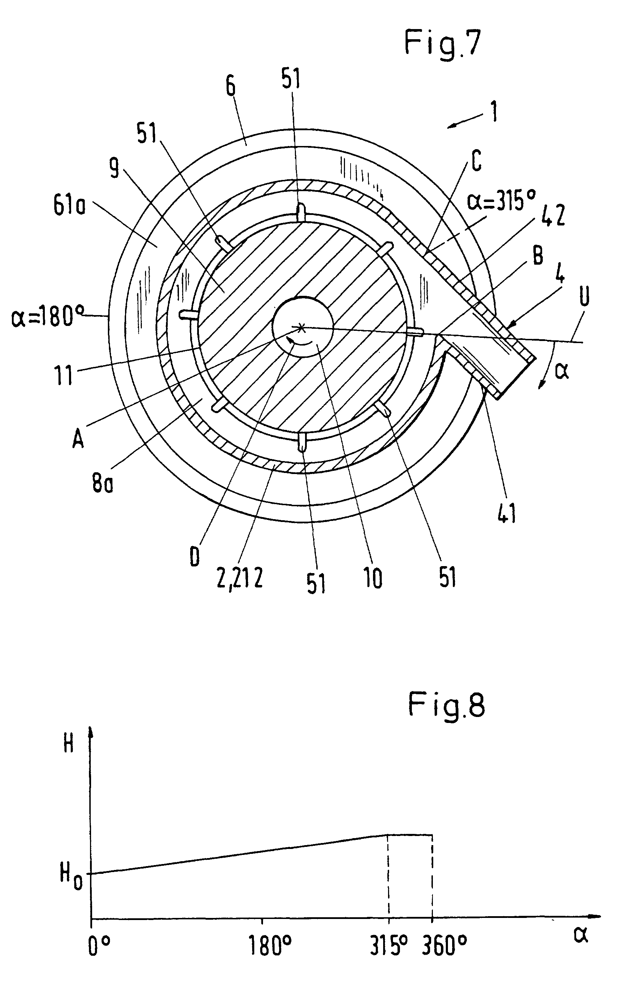

Bei dem zweiten Ausführungsbeispiel nimmt die axiale Höhe H des Leitkanals 8a in Umfangsrichtung zu, sodass der Leitkanal 8a einen axialen Spiralraum bildet. Diese konstruktive Massnahme wird beispielhaft anhand der Fig. 7, die eine der Fig. 3 bzw. Fig. 5 entsprechende Querschnittsdarstellung zeigt, und der Fig. 8 näher erläutert. In Fig. 8 ist ein Diagramm dargestellt, welches die axiale Höhe H des Leitkanals 8a als Funktion eines Azimutwinkels α zeigt. Der Winkel α ist dabei wie folgt festgelegt (siehe Fig. 7). Der Winkel α wird gegen eine gedachte, radial verlaufende Verbindungslinie U zwischen der Rotationsachse A und dem Ansatzpunkt B des kürzeren Schenkels 41 des tangentialen Auslasses 4 gemessen. Diese Verbindungslinie U entspricht also einem Winkel α = 0°. Der Winkel α nimmt in Rotationsrichtung D gesehen zu. Der Ansatzpunkt C des längeren Schenkels 42 des tangentialen Auslasses 4 befindet sich beispielsweise bei dieser konkreten Ausgestaltung bei α = 315°.

Fig. 8 zeigt die axiale Höhe H des Leitkanals 8a als Funktion des Winkels α. Die axiale Höhe H hat für α = 0° ihren minimalen Wert H₀ und nimmt dann mit wachsendem α zu bis zum Ansatzpunkt C des längeren Schenkels 42 bei α = 315°. Entlang des Auslasses 4, das heisst für 315° ≤ α < 360° ist die axiale Höhe H konstant.

Die Ausgestaltung des Pumpengehäuses 8a als axialer Spiralraum ist unter stömungstechnischen Aspekten besonders vorteilhaft, insbesondere in Kombination mit den gewinkelten Flügelblätter 51.

Es versteht sich, dass auch solche Ausgestaltungen möglich sind, bei denen der Leitkanal 8;8a sowohl als radialer als auch als axialer Spiralraum ausgestaltet ist, sich also in Umfangsrichtung betrachtet radial erweitert und gleichzeitig eine zunehmende axiale Höhe H aufweist.

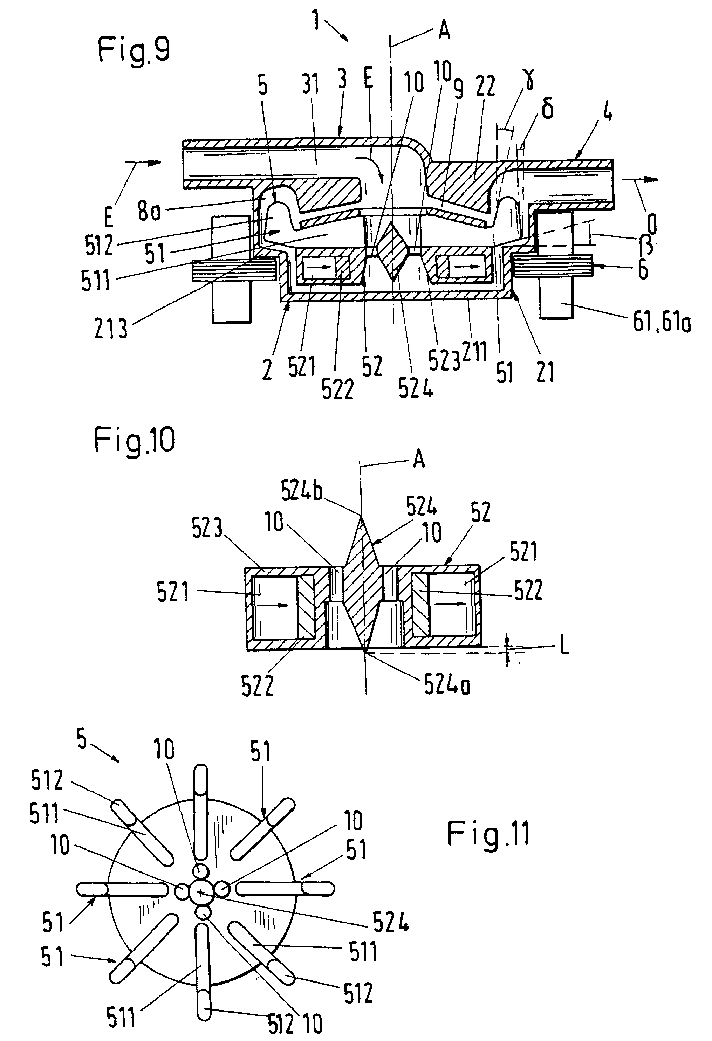

Fig. 9 zeigt in einer Längsschnittdarstellung eine Weiterentwicklung des zweiten Ausführungsbeispiels. Auch in Fig. 9 sind aus Gründen der besseren Übersicht nur zwei der Flügel 51 dargestellt. Ein wesentlicher Unterschied besteht in der Ausgestaltung des Einlasses 3. Der Einlass 3 umfasst bei dieser Weiterentwicklung einen gewinkelten Einlasskanal 31, der sich zunächst im wesentlichen radial zur Rotationsachse A hin erstreckt, und sich dann in die Richtung der Rotationsachse A krümmt, sodass das Fluid aus der axialen Richtung zu dem Flügelrad 5 gelangen kann, wie dies die Pfeile E in Fig. 9 andeuten. Wie dies Fig.9 zeigt, ist der radial verlaufende Teil des Einlasskanals 31 von einer anderen Seite zur Rotationsachse A geführt als die Seite, zu der sich der Auslass 4 erstreckt. Diese Massnahme erlaubt insbesondere in Kombination mit der Ausgestaltung des Leitkanals 8a als axialer Spiralraum eine besonders kompakte Ausgestaltung der Zentrifugalpumpe 1. Da nämlich der Leitkanal 8a gemäss der Darstellung in Fig. 9 auf der linken Seite eine geringere axiale Höhe H aufweist als auf der rechten Seite, wo der Auslass 4 angeordnet ist, kann der radial verlaufende Teil des Einlasskanals 31 bezüglich der axialen Richtung näher am Flügelrad 5 angeordnet werden, insbesondere derart, dass sich der radial verlaufende Teil des Einlasskanals 31 und der Auslass 4 bezüglich ihrere axialen Positionen zumindest teilweise überlappen. Vorzugsweise ist der Einlasskanal 31 in das Pumpengehäuse 2 integriert. Durch diese Massnahmen erhält die Zentrifugalpumpe 1 aufgrund der optimierten Ausnutzung des zur Verfügung stehenden Platzes eine möglichst geringe Ausdehnung in axialer Richtung, wodurch sie sehr kompakt ist und zudem eine hohe Förderleistung ermöglicht.

Eine weitere vorteilhafte Massnahme, die natürlich in sinngemäss gleicher Weise auch für das erste Ausführungsbeispiel bzw. seine Weiterentwicklung Verwendung finden kann, betrifft die Ausgestaltung der Flügel 51 des Flügelrads 5.

Wie aus Fig. 9 ersichtlich sind die radial äusseren Enden der Flügel 51 auf ihrer dem Einlass 3 abgewandten Seite - gemäss der Darstellung in Fig. 9 ist dies die Unterseite der Flügel 51 - jeweils bezüglich der Rotationsebene, die senkrecht zur Rotationsachse A verläuft, unter einem Winkel β abgeschrägt ausgestaltet. Hierdurch wird vermieden, dass die Flügel 51 bei geringfügigen Verkippungen des Flügelrads 5 an das Pumpengehäuse 2, insbesondere den Gehäuseabsatz 213, anschlagen.

Falls die Flügel 51 gefaltet ausgestaltet sind, also einen Axialteil 512 aufweisen, sind vorzugsweise die Axialteile 512 der Flügel 51 an ihren radial inneren und/oder radial äusseren Begrenzungen unter einem Winkel γ bzw. δ abgeschrägt bezüglich der Richtung der Rotationsachse A ausgestaltet, um ein Anschlagen der Flügel 51 bei geringfügigen Verkippungen bezüglich der Rotationsebene zu vermeiden.

Aus praktischen Gründen sind die Winkel β, γ, und δ der Abschrägungen vorzugsweise jeweils kleiner als 10° und speziell kleiner als 5°. Insbesondere sind solche Ausgestaltungen bevorzugt, bei denen alle drei Winkel β, γ und δ den gleichen Wert haben.

Fig. 9 zeigt ferner noch eine ebenfalls bevorzugte Variante für die Ausgestaltung des Rotors 52 des Flügelrads 5. Die Detaildarstellung in Fig. 10 verdeutlicht diese Variante. Im Unterschied zu der weiter vorne beschriebenen Ausgestaltung weist die Ummantelung 523, die den ringförmigen Permanentmagnet 521 sowie den Eisenrückschluss 522 umgibt, in ihrem zentralen Bereich ein doppelkegelförmiges Teil 524 auf, dessen zwei Spitzen 524a und 524b jeweils auf der Rotationsachse A liegen, wobei der eine Kegel des doppelkegelförmigen Teils 524 mit seiner Spitze 524b in Richtung des Einlasses 3 orientiert ist und der andere Kegel mit seiner Spitze 524a vom Einlass 3 weg in Richtung des Gehäusebodens 211 orientiert ist. Der erstgenannte Kegel dient primär einer besseren Flussführung des Fluids. Der andere Kegel ist so ausgestaltet, dass seine Spitze 524a in axialer Richtung um eine Länge L über den Rest der Ummantelung 523 hinausragt. Dadurch weist das Flügelrad 5 an seinem dem Einlass 3 abgewandten Ende in axialer Richtung die vorstehende Spitze 524a auf, mit der es sich notfalls auf dem Boden 211 des Pumpengehäuses 2 abstützen kann. Die Spitze 524a bildet somit ein sogenanntes "Backup-Lager", welches beispielsweise gewährleistet, dass das Flügelrad 5 bei einer Absenkung, die z. B. durch eine kurzfristige Überlastung der passiven magnetischen Axiallagerung auftritt, im wesentlichen unbehindert - nämlich auf der Spitze 524a - weiter rotieren kann.

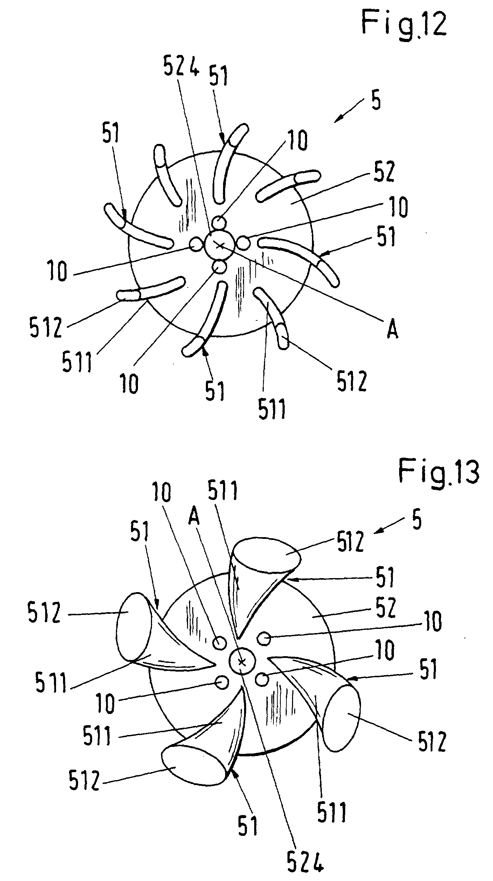

Bei dieser Ausgestaltung des Rotors sind vorzugsweise mehrere Entlastungsbohrungen 10 vorgesehen, die um den doppelkegelförmigen Teil 524 herum angeordnet sind. Dies ist insbesondere aus der Darstellung in Fig. 11 ersichtlich, die eine Aufsicht auf das Flügelrad 5 der in Fig. 9 dargestellten Zentrifugalpumpe 1 vom Einlass 3 her zeigt. Die Deckplatte 9 ist in Fig. 11 nicht dargestellt. Bei dieser konkreten Ausgestaltung sind vier Entlastungsbohrungen 10 vorgesehen, welche den doppelkegelförmigen Teil 524 gleichmässig verteilt umgeben.

Es versteht sich, dass die Ausgestaltung des Rotors 52 bzw. des Flügelrads 5 mit dem doppelkegelförmigen Teil 524 auch bei dem ersten Ausführungsbeispiel bzw. seiner Weiterentwicklung in analoger Weise möglich ist.

Bezüglich der Form der Flügel 51 der erfindungsgemässen Zentrifugalpumpe sind zahlreiche Varianten möglich, von denen hier nur einige nicht abschliessend beschrieben sind. Die Flügel 51 können z. B. wie aus Fig. 2 ersichtlich jeweils geradlinig in radialer Richtung verlaufend ausgestaltet sein. Fig. 12 und Fig. 13, die jeweils eine Aufsicht auf Ausführungsformen für das Flügelrad 5 zeigen (Deckplatte 9 nicht dargestellt), verdeutlichen noch zwei weitere Varianten für die Flügel 51. Bei der in Fig. 12 gezeigten Ausführungsform verlaufen die Flügel 51, speziell ihre Radialteile 511, bezüglich der radialen Richtung gekrümmt nach aussen. Fig. 13 schliesslich zeigt eine Ausführungsform, bei der die Dicke der Flügel nach aussen hin zunimmt.

Es versteht sich, dass die Erfindung nicht auf solche Zentrifugalpumpen beschränkt ist, bei denen das Flügelrad magnetisch gelagert und durch ein Drehfeld angetrieben ist. Die erfindungsgemässe Zentrifugalpumpe eignet sich insbesondere für solche Anwendungen, bei denen ihre äussere Form Beschränkungen unterliegt, wie sie beispielsweise durch die Wickelköpfe 61a verursacht werden.

Die erfindungsgemässe Zentrifugalpumpe zeichnet sich insbesondere durch ihre hohe Leistungsfähigkeit verbunden mit einer äusserst kompakten Ausgestaltung aus.

In der speziellen Ausgestaltung nach dem Prinzip des lagerlosen Motors mit magnetischer, berührungsloser Lagerung des Flügelrads und Antrieb durch ein Drehfeld ist die erfindungsgemässe Zentrifugalpumpe insbesondere für solche Anwendungen geeignet, bei denen eine strikte Isolierung des zu fördernden Fluids von der Umwelt vonnöten ist und bei denen eine Verunreinigung des Fluids - beispielsweise durch Abrieb in mechanischen Lagern - vermieden werden muss. Die erfindungsgemässe Zentrifugalpumpe eignet sich somit insbesondere zum Fördern von biologischen oder hochreinen oder aggressiven bzw. sonst wie gefährlichen Substanzen. Im speziellen ist die erfindungsgemässe Zentrifugalpumpe aufgrund ihrer hohen Förderleistung in Verbindung mit der kompakten Ausgestaltung als Blutpumpe, betrieben ausserhalb oder innerhalb menschlicher oder tierischer Körper, geeignet.

## Patentansprüche

1. Zentrifugalpumpe zum Fördern eines Fluids, insbesondere zum Fördern von Blut, mit einem Pumpengehäuse (2) mit Gehäusedeckel (22), wobei das Pumpengehäuse (2) einen Einlass (3) sowie einen Auslass (4) für das Fluid aufweist, mit einem im Innern des Pumpengehäuses (2) angeordneten Flügelrad (5), das mehrere Flügel (51) aufweist und um eine Rotationsachse (A) rotierbar ist, sowie mit Mitteln zum Antreiben des Flügelrads (5), welche Mittel zum Antreiben einen passiven magnetisch wirksamen Rotor (52) umfassen, der Teil des Flügelrads (5) ist, sowie einen das Pumpengehäuse (2) umgebenden Stator (6), der elektrische Wicklungen (61) aufweist, wobei der Stator (6) als Lager- und Antriebsstator ausgestaltet ist, sodass das Flügelrad (5) im Betriebszustand durch die magnetische Wechselwirkung zwischen dem Stator (6) und dem Rotor (52) sowohl antreibbar als auch berührungslos in dem Pumpengehäuse (2) lagerbar ist, **dadurch gekennzeichnet, dass** der Auslass (4) bezüglich des Flügelrads (5) in axialer Richtung versetzt angeordnet ist wobei in dem Pumpengehäuse (2) ein ringförmiger, in Umfangsrichtung des Pumpengehäuses verlaufenden Leitkanal (8;8a) vorgesehen ist,> der den Einlass (3) mit dem Auslass (4) verbindet, und sich die Flügel (51) des Flügelrads (5) in radialer Richtung gesehen bis in den Leitkanal (8;8a) hinein erstrecken, und eine in axialer Richtung zwischen den Flügeln (51) des Flügelrads (5) und dem Gehäusedeckel (22) des Pumpengehäuses (2) angeordneten Deckplatte (9) vorgesehen ist, die sich in radialer Richtung bis an die radial innere Begrenzung des Leitkanals (8;8a) erstreckt.

2. Zentrifugalpumpe nach Anspruch 1, wobei der im Pumpengehäuse (2) verlaufende Leitkanal (8;8a) den Einlass (3) mit dem Auslass (4) verbindet und der Leitkanal (8;8a) einen Teilraum (81) umfasst, der in axialer Richtung gesehen bezüglich des Flügelrads (5) versetzt angeordnet ist.

3. Zentrifugalpumpe nach einem der vorangehenden Ansprüche, wobei der Rotor (52) im Betriebszustand mittels der elektrischen Wicklungen (61) des Stators (6) bezüglich dreier Freiheitsgrade aktiv ansteuerbar bzw. antreibbar ist und bezüglich dreier weiterer Freiheitsgrade durch Reluktanzkräfte magnetisch passiv im Stator (6) gelagert ist.

4. Zentrifugalpumpe nach einem der vorangehenden Ansprüche, wobei die Flügel (51) des Flügelrads (5) jeweils gewinkelt ausgestaltet sind, sodass sie jeweils einen Radialteil (511) aufweisen, der sich geradlinig oder gekrümmt in radialer Richtung nach aussen erstreckt, sowie einen daran anschliessenden Axialteil (512), der sich im wesentlichen parallel zur Rotationsachse (A) des Flügelrads (5) ersreckt.

5. Zentrifugalpumpe nach einem der vorangehenden Ansprüche, wobei der Leitkanal (8;8a) in radialer Richtung als Spiralraum ausgestaltet ist, sodass der Abstand (d') zwischen den radial äusseren Enden der Flügel (51) und der den Leitkanal (8;8a) begrenzenden Wandung (212) des Pumpengehäuses (2) in Rotationsrichtung (D) gesehen zunimmt.

6. Zentrifugalpumpe nach einem der vorangehenden Ansprüche, wobei die axiale Höhe (H) des Leitkanals (8a) in Umfangsrichtung zunimmt.

7. Zentrifugalpumpe nach einem der vorangehenden Ansprüche, wobei der Einlass (3) einen gewinkelten Einlasskanal (31) umfasst, der sich zunächst im wesentlichen radial zur Rotationsachse (A) hin erstreckt und sich dann in die Richtung der Rotationsachse (A) krümmt, sodass das Fluid aus der axialen Richtung zu dem Flügelrad (5) gelangen kann.

8. Zentrifugalpumpe nach einem der vorangehenden Ansprüche, wobei die radial äusseren Enden der Flügel (51) auf ihrer dem Einlass (3) abgewandten Seite jeweils bezüglich der Rotationsebene abgeschrägt ausgestaltet sind, wobei der Winkel (β) der Abschrägung insbesondere kleiner als 10°, speziell kleiner als 5°, ist.

9. Zentrifugalpumpe nach einem der Ansprüche 4 bis 8, wobei die Axialteile (512) der Flügel (51) an ihren radial inneren und/oder radial äussern Begrenzungen abgeschrägt bezüglich der Richtung der Rotationsachse (A) ausgestaltet sind, wobei der Winkel (γ;δ) der Abschrägung insbesondere kleiner als 10°, speziell kleiner als 5°, ist.

10. Zentrifugalpumpe nach einem der vorangehenden Ansprüche, wobei das Flügelrad (5) in seinem axialen Bereich mindestens eine durchgängige Entlastungsbohrung (10) aufweist.

11. Zentrifugalpumpe nach einem der vorangehenden Ansprüche, wobei das Flügelrad (5) an seinem dem Einlass (3) abgewandten Ende in axialer Richtung eine vorstehende Spitze (524a) aufweist, mit der es sich notfalls auf dem Boden (211) des Pumpengehäuses (2) abstützen kann.

## Revendications

1. Pompe centrifuge pour convoyer un fluide, en particulier pour convoyer du sang, avec un carter de pompe (2) avec un couvercle de carter (22), où le carter de pompe (2) présente une entrée (3) ainsi qu'une sortie (4) pour le fluide, avec une roue à ailettes (5) disposée dans l'intérieur du carter de pompe (2), qui présente plusieurs ailettes (51) et peut tourner autour d'un axe de rotation (A), et avec des moyens pour entraîner la roue à ailettes (5), ces moyens d'entraînement comportant un rotor passif (52), actif magnétiquement qui fait partie de la roue à ailettes (5) ainsi qu'un stator (6) entourant le carter de pompe (2) qui présente des enroulements électriques (61), où le stator (6) est réalisé comme stator de palier et d'entraînement de sorte que la roue à ailettes (5), à l'état de fonctionnement, peut être entraînée par l'interaction magnétique entre le stator (6) et le rotor (52) et peut également être logée sans contact dans le carter de pompe (2), **caractérisée en ce que** la sortie (4) est disposée d'une manière décalée par rapport à la roue à ailettes (5) dans la direction axiale, où est prévu dans le carter de pompe (2) un canal de guidage annulaire (8; 8a) s'étendant dans la direction périphérique du carter de pompe, qui relie l'entrée (3) à la sortie (4), et **en ce que** les ailettes (51) de la roue à ailettes (5), en regardant dans la direction radiale, s'étendent jusque dans le canal de guidage (8; 8a), et **en ce qu'**une plaque de recouvrement (9) disposée dans la direction axiale entre les ailettes (51) de la roue à ailettes (5) et le couvercle de carter (22) du carter de pompe (2) est prévue qui s'étend dans la direction radiale jusqu'à la délimitation radiale interne du canal de guidage (8; 8a).

2. Pompe centrifuge selon la revendication 1, où le canal de guidage (8; 8a) s'étendant dans le carter de pompe (2) relie l'entrée (3) à la sortie (4), et le canal de guidage (8; 8a) comprend une enceinte partielle (81) qui est disposée d'une manière décalée, en regardant dans la direction axiale, par rapport à la roue à ailettes (5).

3. Pompe centrifuge selon l'une des revendications précédentes, où le rotor (52), à l'état de fonctionnement, peut être commandé respectivement entraîné activement au moyen des enroulements électriques (61) du stator (6) relativement à trois degrés de liberté et est logé, relativement à trois autres degrés de liberté, par des forces de réluctance d'une manière magnétiquement passive dans le stator (6).

4. Pompe centrifuge selon l'une des revendications précédentes, où les ailettes (51) de la roue à ailettes (5) sont réalisées chacune de manière angulaire de sorte qu'elles présentent chacune une partie radiale (511) qui s'étend de manière rectiligne ou courbée dans la direction radiale vers l'extérieur, ainsi qu'une partie axiale (512) qui fait suite, qui s'étend sensiblement parallèlement à l'axe de rotation (A) de la roue à ailettes (5).

5. Pompe centrifuge selon l'une des revendications précédentes, où le canal de guidage (8; 8a) est réalisé dans la direction radiale comme enceinte en spirale de sorte que l'écart (d') entre les extrémités radialement externes des ailettes (51) et la paroi (212) du carter de pompe (2) délimitant le canal de guidage (8; 8a) augmente en regardant dans la direction de rotation (D).

6. Pompe centrifuge selon l'une des revendications précédentes, où la hauteur axiale (H) du canal de guidage (8a) augmente dans la direction périphérique.

7. Pompe centrifuge selon l'une des revendications précédentes, où l'entrée (3) comprend un canal d'entrée coudé (31) qui s'étend d'abord sensiblement radialement vers l'axe de rotation (A) et se courbe ensuite dans la direction de l'axe de rotation (A) de sorte que le fluide peut arriver de la direction axiale à la roue à ailettes (5).

8. Pompe centrifuge selon l'une des revendications précédentes, où les extrémités radialement externes des ailettes (51) sont chanfreinées à leur côté éloigné de l'entrée (3) respectivement par rapport au plan de rotation, où l'angle (β) du chanfrein est en particulier plus petit que 10°, en particulier plus petit que 5°.

9. Pompe centrifuge selon l'une des revendications 4 à 8, où les parties axiales (512) des ailettes (51) sont réalisées à leurs délimitations radialement internes et/ou radialement externes d'une manière chanfreinée par rapport à la direction de l'axe de rotation (A), où l'angle (γ; δ) du chanfrein est en particulier plus petit que 10°, en particulier plus petit que 5°.

10. Pompe centrifuge selon l'une des revendications précédentes, où la roue à ailettes (5) présente dans sa zone axiale au moins un perçage de décharge traversant (10).

11. Pompe centrifuge selon l'une des revendications précédentes, où la roue à ailettes (5) présente à son extrémité éloignée de l'entrée (3), dans la direction axiale, une pointe saillante (524a) par laquelle elle peut s'appuyer en cas de besoin sur le fond (211) du carter de pompe (2).

## Claims

1. Centrifugal pump for the forwarding of a fluid, in particular for the forwarding of blood, including a pump housing (2)with a housing cover (22), the pump housing having an inlet (3) and an outlet (4) for the fluid, a vaned wheel (5) which is arranged in the interior of the pump housing (2), which has a plurality of vanes (51) and which can be rotated about an axis of rotation (A), as well as means for driving the vaned wheel (5), said means for driving including a passive, magnetically effective rotor (52) which is part of the vaned wheel (5) and also a stator (6) which surrounds the pump housing (2) and has electrical windings (61), with the stator (6) being designed as a bearing and drive stator so that the vaned wheel (5) can both be driven and journalled without contact in the pump housing (2) by the magnetic interaction between the stator (6) and the rotor (52) in the operating state, **characterised in that** the outlet (4) is arranged offset with respect to the vaned wheel (5) in the axial direction, with a ring-shaped guide passage (8; 8a) which extends in the peripheral direction of the pump housing being provided in the pump housing (2) and connecting the inlet (3) to the outlet (4), with the vanes (51) of the vaned wheel (5) extending into the guide passage (8; 8a) when viewed in the radial direction and with a cover plate (9) which is arranged in the axial direction between the vanes (51) of the vaned wheel (5) and the housing cover (22) of the pump housing and extends in the radial direction approximately up to the radially inner boundary of the guide passage (8; 8a).

2. A centrifugal pump in accordance with claim 1, wherein the guide passage (8; 8a) extending in the pump housing includes a partial space (81) which is arranged offset with respect to the vaned wheel (5) when viewed in the axial direction.

3. A centrifugal pump in accordance with one of the preceding claims, with it being possible to actively excite or drive the rotor (52) in the operating state with respect to three degrees of freedom by means of the electrical windings (61) of the stator (6) and with the rotor (52) being magnetically passively journalled in the stator (6) with respect to three further degrees of freedom by means of reluctance forces.

4. A centrifugal pump in accordance with any one of the preceding claims, with the vanes (51) of the vaned wheel (5) being designed to be angled in each case so that they each have a radial part (511) which extends either rectilinearly or in a curved manner outwardly in the radial direction and an axial part (512) adjoining it which extends substantially parallel to the axis of rotation (A) of the vaned wheel (5).

5. A centrifugal pump in accordance with any one of the preceding claims, with the guide passage (8; 8a) being designed in the radial direction as a spiral space so that the distance (d') between the radial outer ends of the vanes (51) and the wall (212) of the pump housing (2) bounding the guide passage (8; 8a) increases when viewed in the radial direction (D).

6. A centrifugal pump in accordance with any one of the preceding claims, with the axial height (H) of the guide passage (8a) increasing in the peripheral direction.

7. A centrifugal pump in accordance with one of the preceding claims, with the inlet (3) comprising an angled inlet passage (31) which at first extends substantially radially to the axis of rotation (A) and then curves in the direction of the axis of rotation (A) so that the fluid can reach the vaned wheel (5) from the axial direction.

8. A centrifugal pump in accordance with any one of the preceding claims, with the radial outer ends of the vanes (51) being bevelled in each case with respect to the plane of rotation at their ends remote from the inlet (3), with the angle (β) of the bevel in particular being less than 10°, especially less than 5°.

9. A centrifugal pump in accordance with any one of the claims 4 to 8, with the axial parts (512) of the vanes (51) being bevelled at their radially inward and/or radially outward boundaries with respect to the direction of the axis of rotation (A), with the angle (γ; δ) of the bevel in particular being less than 10°, especially less than 5°.

10. A centrifugal pump in accordance with any one of the preceding claims, with the vaned wheel (5) having at least one through-going relief bore (10) in its axial region.

11. A centrifugal pump in accordance with any one of the preceding claims, with the vaned wheel (5) having a projecting tip (524a) at its end remote from the inlet (3) in the axial direction by means of which it can support itself on the base (211) of the pump housing (2) if necessary.
